(19) Europäisches Patentamt
European Patent Office
Office européen des brevets

(11) EP 1 064 927 A1

(12) # DEMANDE DE BREVET EUROPEEN

(43) Date de publication:
**03.01.2001 Bulletin 2001/01**

(51) Int Cl.$^7$: **A61K 7/48**

(21) Numéro de dépôt: **00401473.4**

(22) Date de dépôt: **25.05.2000**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **25.06.1999 FR 9908170**

(71) Demandeur: **L'OREAL**
**75008 Paris (FR)**

(72) Inventeurs:
- **Dubief, Claude**
  **78150 Le Chesnay (FR)**
- **Restle, Serge**
  **95390 Saint-Prix (FR)**
- **Fack, Géraldine**
  **92300 Levallois (FR)**

(74) Mandataire: **Dodin, Catherine**
**L'OREAL-DPI**
**6 rue Bertrand Sincholle**
**92585 Clichy Cédex (FR)**

(54) **Composition cosmétique détergente comprenant une silicone et un polymère amphotère à chaines grasses et utilisation**

(57) L'invention concerne de nouvelles compositions détergentes comprenant, dans un milieu cosmétiquement acceptable, une base lavante, au moins une silicone insoluble dans l'eau et au moins un polymère amphotère comprenant de 1 à 20% en moles d'au moins un monomère de type (méth)acrylate ou (méth)acrylamide ayant au moins une chaîne grasse, ladite chaîne grasse ayant de 8 à 30 atomes de carbone.

Ces compositions possèdent des propriétés cosmétiques améliorées en particulier le lissage des cheveux.

Application au nettoyage, au soin et au coiffage des cheveux.

EP 1 064 927 A1

## Description

La présente invention concerne de nouvelles compositions cosmétiques à propriétés améliorées destinées simultanément au nettoyage, au conditionnement et au coiffage des cheveux, et comprenant, dans un support cosmétiquement acceptable, une base lavante constituée de tensio-actifs à pouvoir détergent dans laquelle sont également présents des silicones en association avec au moins un polymère amphotère comprenant de 1 à 20% en moles d'au moins un monomère de type (méth)acrylate ou (méth)acrylamide ayant au moins une chaîne grasse, ladite chaîne grasse ayant de 8 à 30 atomes de carbone.
L'invention concerne aussi l'utilisation desdites compositions dans l'application cosmétique susmentionnée.

Pour le nettoyage et/ou le lavage des cheveux, l'utilisation de compositions capillaires détergentes (ou shampooings) à base essentiellement d'agents tensio-actifs classiques de type notamment anioniques, non-ioniques et/ou amphothères, mais plus particulièrement de type anioniques, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entrainer à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abimés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les silicones, qui confèrent en effet aux cheveux lavés, secs ou mouillés, une facilité de démélage, une douceur et un lissage nettement accrus par rapport à ce qui peut être obtenu avec les compositions nettoyantes correspondantes qui en sont exemptes.

Toutefois, et malgré les progrès réalisés récemment dans le domaine des shampooings, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoqués ci-avant, de meilleures performances.

La présente invention vise à la satisfaction d'un tel besoin.

Ainsi, à la suite d'importantes recherches menées sur la question, il a maintenant été trouvé par la Demanderesse, de façon totalement inattendue et surprenante, qu'en introduisant au moins un polymère amphotère comprenant de 1 à 20% en moles d'au moins un monomère de type (méth)acrylate ou (méth)acrylamide ayant au moins une chaîne grasse, ladite chaîne grasse ayant de 8 à 30 atomes de carbone, dans des compositions détergentes notamment capillaires contenant des silicones, il est possible d'améliorer de manière substantielle et significative les propriétés cosmétiques attachées à ces dernières, et ceci tout en conservant leur bon pouvoir lavant intrinsèque.

Sans vouloir limiter la présente invention à une quelconque théorie, il semblerait exister lors du rinçage, des interactions et/ou des affinités particulières entre les silicones, les polymères amphotères conformes à l'invention et les cheveux, qui favorisent un dépôt régulier, important et durable desdites silicones et polymères amphotères à la surface desdits cheveux, ce dépôt qualitatif et quantitatif étant probablement l'une des causes de l'amélioration observée au niveau des propriétés finales, en particulier la facilité de coiffage, le maintien, la nervosité et le volume des cheveux traités.
Toutes ces découvertes sont à la base la présente invention.

Ainsi, selon la présente invention, il est maintenant proposé de nouvelles compositions notamment détergentes notamment capillaires, comprenant, dans un milieu cosmétiquement acceptable, une base lavante, au moins une silicone insoluble dans l'eau, et au moins un polymère amphotère comprenant de 1 à 20% en moles d'au moins un monomère de type (méth)acrylate ou (méth)acrylamide ayant au moins une chaîne grasse, ladite chaîne grasse ayant de 8 à 30 atomes de carbone.

L'invention a également pour objet l'utilisation en cosmétique des compositions ci-dessus pour le nettoyage, le conditionnement et le coiffage des cheveux.

Mais d'autres caractéristiques, aspects et avantages de l'invention apparaîtront encore plus clairement à la lecture de la description qui va suivre ainsi que des exemples concrets, mais nullement limitatifs, destinés à l'illustrer.

Comme indiqué précédemment, les constituants essentiels rentrant dans la composition des produits capillaires de l'invention sont (i) le ou les tensioactifs à pouvoir détergent destinés à former la base lavante, (ii) le ou les silicones et (iii) le ou les polymères amphotères particuliers.

A- BASE LAVANTE :

Les compositions conformes à l'invention comprennent nécessairement une base lavante, généralement aqueuse.

Le ou les tensioactifs formant la base lavante peuvent être indifféremment choisis, seuls ou en mélanges, au sein des tensioactifs anioniques, amphotères, non ioniques, zwittérioniques et cationiques. Toutefois, selon l'invention, la base lavante comprend de préférence des tensioactifs anioniques ou des mélanges de tensioactifs anioniques et de tensioactifs amphotères ou de tensioactifs non ioniques.

La quantité minimale de base lavante est celle juste suffisante pour conférer à la composition finale un pouvoir moussant et/ou détergent satisfaisant, et des quantités trop importantes de base lavante n'apportent pas vraiment d'avantages supplémentaires.

Ainsi, selon l'invention, la base lavante peut représenter de 4 % à 50 % en poids, de préférence de 6 % à 25 % en poids, et encore plus préférentiellement de 8 % à 20 % en poids, du poids total de la composition finale.

Les tensioactifs convenant à la mise en oeuvre de la présente invention sont notamment les suivants :

(i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl ($C_6$-$C_{24}$) sulfosuccinates, les alkyl($C_6$-$C_{24}$) éthersulfosuccinates, les alkyl($C_6$-$C_{24}$) amidesulfosuccinates ; les alkyl($C_6$-$C_{24}$) sulfoacétates ; les acyl($C_6$-$C_{24}$) sarcosinates et les acyl($C_6$-$C_{24}$) glutamates. On peut également utiliser les esters d'alkyl($C_6$-$C_{24}$)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl ($C_6$-$C_{24}$) éther carboxyliques polyoxyalkylénés, les acides alkyl($C_6$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'alkylène en particulier d'éthylène, et leurs mélanges.

Les tensioactifs anioniques comportant un groupement carboxyliques sont particulièrement préférés.

(ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. On notera que les alkylpolyglycosides constituent des tensio-actifs non-ioniques rentrant particulièrement bien dans le cadre de la présente invention.

(iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

$$R_2\text{-CONHCH}_2\text{CH}_2\text{-N}(R_3)(R_4)(\text{CH}_2\text{COO}^-) \quad (2)$$

dans laquelle : $R_2$ désigne un radical alkyle d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ; et

$$R_2'\text{-CONHCH}_2\text{CH}_2\text{-N(B)(C)} \quad (3)$$

dans laquelle :
B représente $-CH_2CH_2OX'$, C représente $-(CH_2)_z$ -Y', avec z = 1 ou 2,
X' désigne le groupement $-CH_2CH_2$-COOH ou un atome d'hydrogène
Y' désigne -COOH ou le radical $-CH_2$- CHOH - $SO_3H$
$R_2'$ désigne un radical alkyle d'un acide $R_9$ -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.

Dans les compositions conformes à l'invention, on utilise de préférence des mélanges d'agents tensioactifs et en particulier des mélanges d'agents tensioactifs anioniques et d'agents tensioactifs amphotères ou non ioniques. Un mélange particulièrement préféré est un mélange constitué d'au moins un agent tensioactif anionique carboxylique et d'au moins un agent tensioactif amphotère ou non ionique.

On utilise de préférence un agent tensioactif anionique choisi parmi les acides alkyl ($C_6$-$C_{24}$) éther carboxyliques polyoxyalkylénés, les acides alkyl($C_6$-$C_{24}$)aryl éther carboxyliques polyoxyalkylénés, les acides alkyl($C_6$-$C_{24}$) amido éther carboxyliques polyoxyalkylénés et leur mélange avec un tensioactif sulfoné ou sulfatés tels les alkyl($C_{12}$-$C_{14}$) sulfates de sodium, de triéthanolamine ou d'ammonium, les alkyl ($C_{12}$-$C_{14}$)éthersulfates de sodium oxyéthylénés à 2,2 moles d'oxyde d'éthylène, le cocoyl iséthionate de sodium et leurs mélange avec :

- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL® C2M CONC" en solution aqueuse à 38 % de matière active ou sous la dénomination MIRANOL® C32;

- soit un agent tensioactif amphotère de type zwittérionique tel que les alkylbétaïnes en particulier la cocoylbétaïne commercialisée sous la dénomination "DEHYTON® AB 30" en solution aqueuse à 32 % de MA par la société HENKEL ou les alkylamidoalkylbétaïnes telles que la TEGOBETAINE® F50 commercialisée par la société GOLDSCHMIDT.

- soit un agent tensioactif non ionique de type alkylpoluglucoside.

(iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.
On notera que les tensioactifs cationiques, dont l'utilisation n'est pas exclue, ne constituent pas des tensioactifs préférés pour la mise en oeuvre de la présente invention.

B- SILICONE(S) :

Les compositions conformes à l'invention comprennent en outre nécessairement une silicone insoluble dans l'eau.
Les silicones utilisables conformément à l'invention peuvent être en particulier des polyorganosiloxanes insolubles dans la composition. Ces silicones peuvent se présenter sous forme d'huiles, de cires, de résines ou de gommes.
La viscosité des silicones est mesurée à 25°C selon la norme ASTM 445 Appendice C.
Les silicones insolubles dans l'eau sont insolubles dans l'eau à une concentration supérieure ou égale à 0,1% en poids dans l'eau à 25°C, c'est à dire qu'elles ne forment pas une solution isotrope transparente.
Les organopolysiloxanes sont définis plus en détail dans l'ouvrage de Walter NOLL "Chemistry and Technology of Silicones" (1968) Academie Press.
On utilise de préférence des silicones non volatiles et plus particulièrement

(i) les polyalkylsiloxanes ;
(ii) les polyarylsiloxanes ;
(iii) les polyalkylarylsiloxanes ;
(iv) les gommes de silicone ;
(v) les résines de silicone ;
(vi) ou leurs mélanges.

Les polyalkylsiloxanes ont de préférence une viscosité supérieure ou égale à 500 cSt et inférieure à 1.000.000, plus particulièrement comprise entre 10.000 et 700.000 cSt. Parmi les polyalkylsiloxanes, on peut citer principalement :

- les polydiméthylsiloxanes linéaires à groupements terminaux triméthylsilyle, comme par exemple, et à titre non limitatif, les huiles SILBIONE® de la série 70047 commercialisées par RHODIA CHIMIE, l'huile SILBIONE® 47 V 500 000 de RHODIA CHIMIE ou certaines VISCASIL de la GENERAL ELECTRIC ;
- les polydiméthylsiloxanes linéaires à groupements terminaux hydroxydiméthylsilyle telles que les huiles de la série 48 V de RHODIA CHIMIE.

Dans cette classe de polyalkylsiloxanes, on peut également mentionner les polyalkylsiloxanes vendus par la société GOLDSCHMIDT sous les dénominations commerciales ABILWAX® 9800 et ABILWAX® 9801 qui sont des polyalkyl($C_1$-$C_{20}$)siloxanes.
Parmi les polyalkylarylsiloxanes, on peut citer les polydiméthylméthylphénylsiloxanes ou les polydiméthyldiphénylsiloxanes, linéaires ou ramifiés tels que le produit DC 556 COSMETIC GRAD FLUID de DOW CORNING.
Les gommes de silicone, conformes à l'invention, sont des polyorganosiloxanes de masse moléculaire moyenne en nombre comprise entre 200.000 et 1.000.000, utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.
On cite, par exemple, les composés suivants :

- polydiméthylsiloxane,
- poly[(diméthylsiloxane)/(méthylvinylsiloxane)],
- poly[(diméthylsiloxane)/(diphénylsiloxane)],
- poly[(diméthylsiloxane)/(phénylméthylsiloxane)],
- poly[(diméthylsiloxane)/(diphénylsiloxane)/(méthylvinylsiloxane)],

On peut citer, par exemple, les mélanges suivants :

1) les mélanges formés à partir d'un polydiméthylsiloxane hydroxylé en bout de chaîne (DIMETHICONOL selon la nomenclature CTFA) et d'un polydiméthylsiloxane cyclique (CYCLOMETHICONE selon la nomenclature CTFA) tels que le produit Q2 1401 vendu par la société DOW CORNING ;

2) les mélanges formés à partir d'une gomme de polydiméthylsiloxane avec une silicone cyclique tels que le produit SF 1214 SILICONE FLUID de GENERAL ELECTRIC qui est une gomme SE 30 de poids moléculaire 500.000 solubilisée dans la SF 1202 SILICONE FLUID (décaméthylcyclopentasiloxane) ;

3) les mélanges de deux polydiméthylsiloxanes (PDMS) de viscosité différente, notamment d'une gomme PDMS et d'une huile PDMS tels que les produits SF 1236 et CF 1241 de la GENERAL ELECTRIC. Le produit SF 1236 est le mélange d'une huile SE 30 définie ci-dessus de viscosité 20 $m^2/s$ et d'une huile SF 96 de viscosité $5.10^{-5} m^2/s$ (15% de gomme SE 30 et 85% d'huile SF 96). Le produit CF 1241 est le mélange d'une gomme SE 30 (33%) et d'une PDMS (67%) de viscosité $10^{-3}$ $m^2/s$.

Les résines de silicone conformes à l'invention sont de préférence des systèmes siloxaniques réticulés renfermant les unités :
$R_2SiO_{2/2}$, $RSiO_{3/2}$, $SiO_{4/2}$, dans lesquelles R désigne un groupe hydrocarboné possédant 1 à 6 atomes de carbone ou un groupement phényle. Parmi ces produits, ceux particulièrement préférés sont ceux où R désigne un radical alkyle inférieur ou phényle.
Parmi ces résines, on peut citer le produit vendu sous le nom DOW CORNING 593 par DOW CORNING ou ceux vendus sous le nom SILICONE FLUID SS 4267 par la GENERAL ELECTRIC et qui sont des diméthyl/triméthypolylsiloxanes.
Selon l'invention, toutes les silicones peuvent également être utilisées sous forme d'émulsions ou de microémulsions.
Les silicones particulièrement préférées conformément à l'invention sont :

- les silicones choisies dans la famille des polydiméthylsiloxanes à groupements terminaux triméthylsilyle telles que les huiles ayant une viscosité comprise entre 0,2 et 2,5 $m^2/s$ à 25° C telles que les huiles de la séries DC200 de DOW CORNING en particulier celle de viscosité 60 000 cSt, des séries SILBIONE® 70047 et 47 et plus particulièrement l'huile SILBIONE® 70 047 V 500 000 commercialisées par la société RHODIA CHIMIE, ou l'huile de silicone AK 300.000 de la société WACKER, les polydiméthylsiloxanes à groupements terminaux diméthylsilanol tels que les diméthiconol;

- les mélanges d'organopolysiloxanes et de silicones cycliques tels que le produit Q2 1401 commercialisé par la société DOW CORNING, et le produit SF 1214 commercialisé par la société GENERAL ELECTRIC ;

- les mélanges de deux PDMS de viscosités différentes notamment d'une gomme et d'une huile tels que le produit SF 1236 commercialisé par la société GENERAL ELECTRIC ;

- la résine d'organopolysiloxane commercialisée sous la dénomination DOW CORNING 593;

Selon l'invention, la ou les silicones peuvent représenter de 0,001 % à 10 % en poids, de préférence de 0,005 % à 5 % en poids, et encore plus préférentiellement de 0,01 % à 3 % en poids, du poids total de la composition finale.

<u>C- Polymères amphotères comprenant au moins une chaîne grasse</u> :

Selon une caractéristique essentielle des compositions détergentes notamment capillaires conformes à l'invention, ces dernières contiennent en outre au moins un polymère amphotère comprenant au moins une chaîne grasse.
Les polymères amphotères selon l'invention comprennent de 1 à 20 % en moles de monomère comportant une chaîne grasse, et de préférence de 1,5 à 15 % en moles et encore plus particulièrement de 1,5 à 6% en moles par rapport au nombre total de moles de monomères.
Les polymères amphotères selon l'invention peuvent résulter de la copolymérisation

1) d'au moins un monomère de formule (Ia) ou (Ib):

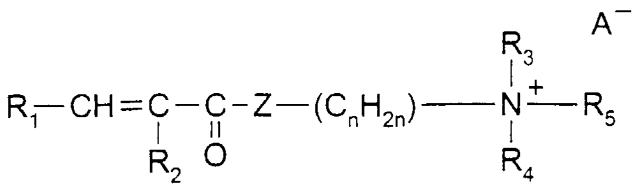 (Ia)

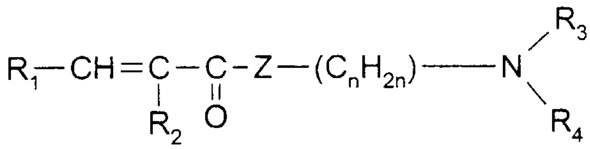 (Ib)

dans lesquelles $R_1$ et $R_2$, identiques ou différents, représentent un atome d'hydrogène ou un radical méthyle, $R_3$, $R_4$ et $R_5$, identiques ou différents, représente un radical alkyle linéaire ou ramifié ayant de 1 à 30 atomes de carbone,
Z représente un groupe NH ou un atome d'oxygène,
n est un nombre entier de 2 à 5,
$A^-$ est un anion issu d'un acide organique ou minéral, tel qu'un anion méthosulfate ou un halogénure tel que chlorure ou bromure.
2) d'au moins un monomère de formule (II)

$$R_6-CH=CR_7-COOH \qquad (II)$$

dans laquelle $R_6$ et $R_7$, identiques ou différents, représentent un atome d'hydrogène ou un radical méthyle; et
3) d'au moins un monomère de formule (III):

$$R_6-CH=CR_7-COXR_8 \qquad (III)$$

dans laquelle $R_6$ et $R_7$, identiques ou différents, représentent un atome d'hydrogène ou un radical méthyle, X désigne un atome d'oxygène ou d'azote et $R_8$ désigne un radical alkyle linéaire ou ramifié ayant de 1 à 30 atomes de carbone;

l'un au moins des monomères de formule (Ia), (Ib) ou (III) comportant au moins une chaîne grasse ayant de 8 à 30 atomes de carbone.

Les monomères de formule (Ia) et (Ib) de la présente invention sont choisis, de préférence, dans le groupe constitué par :

- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,

ces monomères étant éventuellement quaternisés par exemple par un halogénure d'alkyle en $C_1$-$C_4$ ou un sulfate de dialkyle en $C_1$-$C_4$.

Plus particulièrement, le monomère de formule (Ia) est choisi parmi le chlorure d'acrylamidopropyl triméthyl ammonium et le chlorure de méthacrylamidopropyl triméthyl ammonium.

Les monomères de formule (II) de la présente invention sont choisis, de préférence, dans le groupe constitué par l'acide acrylique, l'acide méthacrylique, l'acide crotonique et l'acide méthyl-2 crotonique.
Plus particulièrement, le monomère de formule (II) est l'acide acrylique.

Les monomères de formule (III) de la présente invention sont choisis, de préférence, dans le groupe constitué des acrylates ou méthacrylate d'alkyle en $C_{12}$-$C_{22}$ et plus particulièrement en $C_{16}$-$C_{18}$.

Les monomères constituant les polymères amphotères de l'invention sont de préférence déjà neutralisés et/ou quaternisés.

Le rapport du nombre de charges cationiques/charges anioniques est de préférence égal à environ 1.

Les poids moléculaires moyen en poids des polymères amphotères selon l'invention peuvent varier de 500 à 50.000.000 et sont de préférence compris entre 10.000 et 5 000 000.

Les polymères selon l'invention peuvent également contenir d'autre monomères tels que des monomères non ioniques et en particulier tels que les acrylates ou méthacrylates d'alkyle en $C_1$-$C_4$.

Les polymères amphotères selon l'invention sont notamment décrits dans la demande de brevet WO9844012.

Les polymères amphotères particulièrement préférés selon l'invention sont choisis parmi les copolymères acide acrylique/chlorure d'acrylamidopropyl triméthyl ammonium/méthacrylate de stéaryle.

Le polymère amphotère est utilisé de préférence en une quantité comprise entre 0,05 et 10% en poids par rapport au poids total de la composition. Plus préférentiellement, cette quantité est comprise entre 0,1 et 5% en poids par rapport au poids total de la composition.

Le milieu aqueux cosmétiquement acceptable peut être constitué uniquement par de l'eau ou par un mélange d'eau et d'un solvant cosmétiquement acceptable tel qu'un alcool inférieur en $C_1$-$C_4$, comme l'éthanol, l'isopropanol, le tertiobutanol, le n-butanol ; les alkylèneglycols comme le propylèneglycol, les éthers de glycols.
De préférence , la composition comprend de 50 à 95 % en poids d'eau par rapport au poids total de la composition.

Les compositions détergentes selon l'invention présentent un pH final généralement compris entre 3 et 10. De préférence, ce pH est compris entre 4 et 9. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en $C_{10}$-$C_{30}$ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les alcools gras supérieurs à $C_{16}$, les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions détergentes selon l'invention peuvent bien entendu contenir en outre tous les adjuvants usuels rencontrés dans le domaine des shampooings, comme par exemple des parfums, des agents conservateurs, des sequestrants, des épaississants, des adoucissants, des modificateurs de mousse, des colorants, des agents nacrants, des agents hydratants, des agents anti-pelliculaires ou anti-séborrhéiques, des filtres solaires et autres.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les agents tensioactifs cationiques, les polymères anioniques ou non ioniques et plus particulièrement cationiques, les polymères amphotères différents de ceux de l'invention, les protéines, les hydrolysats de protéines, les céramides, les pseudocéramides, les acides gras à chaînes linéaires ou ramifiées en $C_{16}$-$C_{40}$ tels que l'acide méthyl-18 eicosanoique, les hydroxyacides, les vitamines, le panthénol, les silicones, volatiles ou non volatiles, solubles et insolubles dans le milieu, les huiles végétales, les huiles de synthèses et leurs mélanges.

Les compositions selon l'invention comprennent de préférence un ou plusieurs polymères cationiques.
Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Parmi tous les polymères cationiques susceptibles d'être utilisés dans le cadre de la présente invention, on

préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL, les copolymères vinylpyrrolidone / méthacrylamidopropyl dimethylamine et leurs mélanges.

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

Les compositions selon l'invention peuvent contenir également des synergistes de mousses tels que des 1,2-alcanediols en $C_{10}$-$C_{18}$ ou des alcanolamides gras dérivés de mono ou de diéthanolamine.

Bien entendu, l'homme du métier veillera à choisir ce ou ces éventuels composés complémentaires et/ou leurs quantités de manière telle que les propriétés avantageuses attachées intrinsèquement à l'association conforme à l'invention ne soient pas, ou substantiellement pas, altérées par la ou les adjonctions envisagées.

Ces compositions peuvent se présenter sous la forme de liquides plus ou moins épaissis, de crèmes ou de gel et elles conviennent principalement au lavage, au soin et/ou le coiffage des cheveux.

Les compositions de l'invention peuvent encore se présenter sous la forme de compositions lavantes pour la peau, et en particulier sous la forme de solutions ou de gels pour le bain ou la douche ou de produits démaquillants.

Lorsque les compositions conformes à l'invention sont mises en oeuvre comme des shampooings classiques, elles sont simplement appliquées sur les matières kératiniques en particulier les cheveux secs ou de préférence mouillés et la mousse générée par massage ou friction avec les mains est ensuite éliminée, après un éventuel temps de pause, par rinçage de préférence à l'eau, l'opération pouvant être répétée une ou plusieurs fois.

L'invention a également pour objet un procédé de lavage et de conditionnement des matières kératiniques consistant à appliquer sur lesdites matières sèches ou mouillées une quantité efficace d'une composition telle que définie ci-dessus, puis à effectuer un rinçage à l'eau après un éventuel temps de pause.

Des exemples concrets, mais nullement limitatifs, illustrant l'invention vont maintenant être donnés.

## EXEMPLE 1

On a réalisé deux compositions de shampooing, l'une conforme à l'invention (composition A) et l'autre comparative (composition B), différant l'une de l'autre simplement par la nature du polymère amphotère employé :

| | A | B |
|---|---|---|
| Lauryléther sulfate de sodium (2,2 OE) | 15,4 g MA | 15,4 g MA |
| Sodium Cocoamphocarboxyglycinate (MIRANOL C2M Conc de RHODIA CHIMIE) | 3 gMA | 3 gMA |
| Polydiméthylsilaxane de viscosité 500.000 cSt (MIRASIL DM 500.000 de RHODIA, CHIMIE) | 2,7 g | 2,7 g |
| Terpolymère de chlorure de méthacrylamidopropyl triméthyl ammonium, d'acide acrylique et de méthacrylate de stéaryle (49/49/2 en %molaire) | 0,5 g MA | |
| Terpolymère de chlorure de méthacrylamidopropyl triméthyl ammonium, d'acide acrylique (50/50 en % molaire) | | 0,5 g MA |
| 1-(Hexadécyloxy) - 2 - octadécanol / alcool cétylique | 2,5 | 2,5 |
| Conservateur | q.s | q.s |
| pH ajusté à | 7 | 7 |
| Eau q.s.p | 100 g | 100 g |

Des mèches de 2,5 g de cheveux naturels d'une longueur de 27 cm sont préalablement humidifiées puis mises en contact avec 1 g de la composition A selon l'invention pendant 5 minutes puis rincées à l'eau. Les mèches sont ensuite séchées sous un casque pendant 20 minutes à 65°C. On procède selon le même mode opératoire que ci-dessus avec la composition comparative B.

Evaluation du toucher des mèches.

On présente à un panel de 10 testeurs les mèches préparées comme indiqué ci-dessus. On leur demande d'indiquer les mèches qu'ils jugent plus douces et lisses. Les 10 juges sont unanimes et déclarent que les cheveux traités à l'aide de la composition A sont plus doux et lisses.

## Revendications

1. Composition détergente caractérisée par le fait qu'elle comprend, dans un milieu cosmétiquement acceptable, une base lavante, au moins une silicone insoluble dans l'eau, au moins un polymère amphotère comprenant de 1 à 20% en moles d'au moins un monomère de type (méth)acrylate ou (méth)acrylamide ayant au moins une chaîne grasse, ladite chaîne grasse ayant de 8 à 30 atomes de carbone.

2. Composition selon la revendication 1, caractérisée par le fait que ladite base lavante comprend un ou plusieurs tensioactifs choisis parmi des tensioactifs anioniques, amphotères, non ioniques, zwittérioniques et leurs mélanges.

3. Composition selon la revendication 1 ou 2, caractérisée par le fait que ladite base lavante est présente à une teneur pondérale comprise entre 4 % et 50 % par rapport au poids total de la composition, de préférence comprise entre 6 % et 25 %.

4. Composition selon l'une quelconque des revendications 1 à 3, caractérisée par le fait que ledit polymère amphotères comprend de 1,5 à 15 moles% de monomère comportant une chaîne grasse, et de préférence de 1,5 à 6 moles % par rapport au nombre total de moles de monomères.

5. Composition selon l'une quelconque des revendications 1 à 4, caractérisée par le fait les polymères amphotères résultent de la copolymérisation

   1) d'au moins un monomère de formule (Ia) ou (Ib):

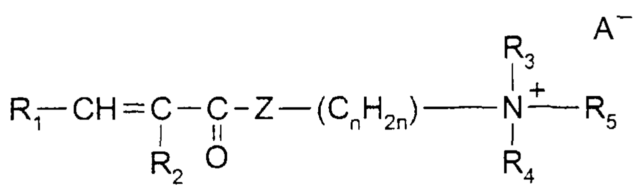

(Ia)

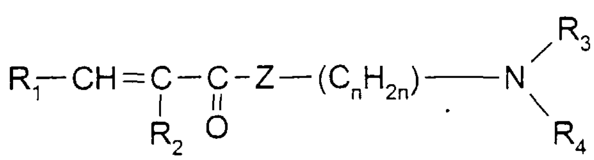

(Ib)

dans lesquelles $R_1$ et $R_2$, identiques ou différents, représentent un atome d'hydrogène ou un radical méthyle,
$R_3$, $R_4$ et $R_5$, identiques ou différents, représente un radical alkyle linéaire ou ramifié ayant de 1 à 30 atomes de carbone,
Z représente un groupe NH ou un atome d'oxygène,
n est un nombre entier de 2 à 5,
$A^-$ est un anion issu d'un acide organique ou minéral.
2) d'au moins un monomère de formule (II)

$$R_6-CH=CR_7-COOH \qquad (II)$$

dans laquelle $R_6$ et $R_7$, identiques ou différents, représentent un atome d'hydrogène ou un radical méthyle; et

3) d'au moins un monomère de formule (III):

$$R_6-CH=CR_7-COXR_8 \qquad (III)$$

dans laquelle $R_6$ et $R_7$, identiques ou différents, représentent un atome d'hydrogène ou un radical méthyle, X désigne un atome d'oxygène ou d'azote et $R_8$ désigne un radical alkyle linéaire ou ramifié ayant de 1 à 30 atomes de carbone;

l'un au moins des monomères de formule (Ia), (Ib) ou (III) comportant au moins une chaîne grasse ayant de 8 à 30 atomes de carbone.

**6.** Composition selon la revendications 5, caractérisée par le fait que les monomères de formule (Ia) et (Ib) de la présente invention sont choisis dans le groupe constitué par :

- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide, ces monomères étant éventuellement quaternisés

**7.** Composition selon l'une quelconque des revendications 5 ou 6, caractérisée par le fait que le monomère de formule (Ia) est choisi parmi le chlorure d'acrylamidopropyl triméthyl ammonium et le chlorure de méthacrylamidopropyl triméthyl ammonium.

**8.** Composition selon l'une quelconque des revendications 5 à 7, caractérisée par le fait que les monomères de formule (II) sont choisis dans le groupe constitué par l'acide acrylique, l'acide méthacrylique, l'acide crotonique et l'acide méthyl-2 crotonique.

**9.** Composition selon l'une quelconque des revendications 5 à 8, caractérisée par le fait que les monomères de formule (III) sont choisis dans le groupe constitué des acrylates ou méthacrylate d'alkyle en $C_{12}$-$C_{22}$ et plus particulièrement en $C_{16}$-$C_{18}$.

**10.** Composition selon l'une quelconque des revendications 1 à 9, caractérisée par le fait que lesdits polymères amphotères sont choisis parmi les copolymères acide acrylique/chlorure d'acrylamidopropyl triméthyl ammonium/ méthacrylate de stéaryle.

**11.** Composition selon l'une quelconque des revendications précédentes, caractérisée par le fait que ledit polymère amphotère est utilisé dans les compositions selon l'invention en une quantité comprise entre 0,05 et 10% en poids par rapport au poids total de la composition.

**12.** Composition selon l'une quelconque des revendications 1 à 11, caractérisée par le fait que lesdites silicones sont choisies dans le groupe constitué par :

(i) les polyalkylsiloxanes ;
(ii) les polyarylsiloxanes ;
(iii) les polyalkylarylsiloxanes ;
(iv) les gommes de silicone ;
(v) les résines de silicone ;
(vi) ou leurs mélanges.

**13.** Composition selon l'une quelconque des revendications 1 à 12, caractérisée par le fait que lesdites silicones sont choisies parmi :

les polydiméthylsiloxanes à groupements terminaux triméthylsilyle

les polydiméthylsiloxanes à groupements terminaux diméthylsilanol.

**14.** Compositions selon l'une quelconque des revendications précédentes, caractérisées par le fait que ladite silicone est présente à une teneur pondérale comprise entre 0,01 % et 10 % par rapport au poids total de la composition et de préférence comprise entre 0,05 % et 5 %.

**15.** Composition selon l'une quelconque des revendications précédentes, caractérisée par le fait qu'elle comprend en outre au moins un additif choisi parmi les agents tensioactifs cationiques, les polymères anioniques ou non ioniques et plus particulièrement cationiques, les polymères amphotères différents de ceux de l'invention, les protéines, les hydrolysats de protéines, les céramides, les pseudocéramides, les acides gras à chaînes linéaires ou ramifiées en $C_{16}$-$C_{40}$ tels que l'acide méthyl-18 eicosanoique, les hydroxyacides, les vitamines, le panthénol, les silicones, volatiles ou non volatiles, solubles et insolubles dans le milieu, les huiles végétales, les huiles de synthèses et leurs mélanges.

**16.** Composition selon la revendication précédente, caractérisée par le fait que ledit polymère cationique est choisi parmi les dérivés d'éther de cellulose quaternaires, les cyclopolymères, les polysaccharides cationiques, les copolymères vinylpyrrolidone / méthacrylamidopropyl dimethylamine et leurs mélanges.

**17.** Composition selon l'une quelconque des revendications précédentes, caractérisée par le fait qu'elles présentent un pH compris entre 4 et 9.

**18.** Utilisation d'une composition telle définie à l'une quelconque des revendications précédentes pour le nettoyage et/ou le soin et/ou le conditionnement et/ou le coiffage des cheveux.

**19.** Procédé de lavage et de conditionnement des matières kératiniques consistant à appliquer sur lesdites matières sèches ou mouillées une quantité efficace d'une composition telle que définie à l'une quelconque des revendications 1 à 17, puis à effectuer un rinçage à l'eau après un éventuel temps de pause.

Office européen des brevets

# RAPPORT DE RECHERCHE EUROPEENNE

Numéro de la demande
EP 00 40 1473

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | DATABASE WPI<br>Section Ch, Week 199417<br>Derwent Publications Ltd., London, GB;<br>Class A96, AN 1994-140934<br>XP002135709<br>& JP 06 087721 A (KAO CORP),<br>29 mars 1994 (1994-03-29)<br>* abrégé *<br>--- | 1-19 | A61K7/48 |
| X | WO 99 13842 A (YANG JIAN ZHONG ;MITSUMATSU ARATA (JP); PROCTER & GAMBLE (US))<br>25 mars 1999 (1999-03-25)<br>* page 10, ligne 27 - page 11, ligne 11 *<br>* page 24, ligne 7 - page 30, ligne 3 *<br>--- | 1-5, 10-19 | |
| D,A | WO 98 44012 A (CALGON CORP)<br>8 octobre 1998 (1998-10-08)<br>* le document en entier *<br>--- | 1-11, 15-19 | |
| A | EP 0 272 472 A (WELLA AG)<br>29 juin 1988 (1988-06-29)<br>* revendications *<br>--- | 1-5, 15-19 | DOMAINES TECHNIQUES RECHERCHES (Int.Cl.7) |
| | | | A61K |
| T | FR 2 779 642 A (OREAL)<br>17 décembre 1999 (1999-12-17)<br>* revendications 1,15,16 *<br>----- | 1-4,11, 14-19 | |

Le présent rapport a été établi pour toutes les revendications

| Lieu de la recherche | Date d'achèvement de la recherche | Examinateur |
|---|---|---|
| LA HAYE | 31 octobre 2000 | Couckuyt, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 00 40 1473

La présente annexe indique les membres de la famille de brevets relatifs aux documents brevets cités dans le rapport de recherche européenne visé ci–dessus.
Lesdits members sont contenus au fichier informatique de l'Officeeuropéen des brevets à la date du
Les renseignements fournis sont donnés à titre indicatif et n'engagent pas la responsabilité de l'Office européen des brevets.

31-10-2000

| Document brevet cité au rapport de recherche | Date de publication | Membre(s) de la famille de brevet(s) | Date de publication |
|---|---|---|---|
| JP 6087721 A | 29-03-1994 | AUCUN | |
| WO 9913842 A | 25-03-1999 | AU 4416097 A | 05-04-1999 |
| WO 9844012 A | 08-10-1998 | US 5879670 A | 09-03-1999 |
| | | AU 6742998 A | 22-10-1998 |
| | | BR 9809025 A | 01-08-2000 |
| | | EP 1021471 A | 26-07-2000 |
| | | PL 337609 A | 28-08-2000 |
| | | US 6066315 A | 23-05-2000 |
| EP 0272472 A | 29-06-1988 | DE 3644097 A | 07-07-1988 |
| | | AT 66364 T | 15-09-1991 |
| | | CA 1294613 A | 21-01-1992 |
| | | DE 3772353 A | 26-09-1991 |
| | | DK 680687 A | 24-06-1988 |
| | | ES 2026172 T | 16-04-1992 |
| | | GR 88300162 T | 31-01-1989 |
| | | GR 3003157 T | 17-02-1993 |
| | | US 4938950 A | 03-07-1990 |
| FR 2779642 A | 17-12-1999 | AU 3391899 A | 23-12-1999 |
| | | BR 9902417 A | 30-05-2000 |
| | | CN 1243699 A | 09-02-2000 |
| | | CZ 9902032 A | 15-12-1999 |
| | | EP 0966946 A | 29-12-1999 |
| | | JP 2000007532 A | 11-01-2000 |
| | | PL 333698 A | 20-12-1999 |

EPO FORM P0460

Pour tout renseignement concernant cette annexe : voir Journal Officiel de l'Office européen des brevets, No.12/82